# FASCICULE DE BREVET EUROPEEN

(11) **EP 4 124 432 B1**
(45) Date de publication et mention de la délivrance du brevet: **10.09.2025**
(21) Numéro de dépôt: 22187545.3
(22) Date de dépôt: 28.07.2022
(51) Int. Cl.: B27H 3/00, B27H 3/02, B27H 3/04, B27H 5/00, B27H 5/02, B27H 5/12, A61L 2/025

(54) **PROCÉDÉ DE RECONDITIONNEMENT DE BARRIQUES PAR ULTRASONS ET BARRIQUE OBTENUE SELON UN TEL PROCÉDÉ**
VERFAHREN ZUR REKONDITIONIERUNG VON FÄSSERN DURCH ULTRASCHALL UND NACH EINEM SOLCHEN VERFAHREN HERGESTELLTE FÄSSER
PROCESS FOR RECONDITIONING BARRELS USING ULTRASOUND, AND BARREL OBTAINED USING SUCH A PROCESS

(30) Priorité: 29.07.2021 FR 2108258
(43) Date de publication de la demande: 01.02.2023
(73) Titulaire: TSO TONNELLERIES, 81600 Gaillac (FR)
(72) Inventeur: DE MONTGOLFIER, Baudouin, 81600 Gaillac (FR)
(74) Mandataire: Ipside

(56) Documents cités:
- WO-A1-2005/039788
- FR-A1- 2 975 312
- FR-A1- 3 013 616
- US-A1- 2006 191 424

## Description

### Domaine technique

La présente invention concerne un procédé de reconditionnement de tonneau usagé selon le préambule de la revendication 1. Un tel procédé et le tonneau correspondant sont connus selon le document US 2006/191424 A1.

### Etat de l'art

Les tonneaux sont connus depuis l'antiquité, avec le cintrage du bois à chaud. Ce contenant très pratique fut utilisé et continue d'être utilisé de nos jours pour la conservation ou l'élevage ainsi que le transport de divers produits alimentaires, surtout liquides, dont le vin principalement.

Malgré les importantes avancées dans les technologies alimentaires, et plus spécifiquement vinicoles, le remplacement du tonneau traditionnel en bois parait difficilement envisageable. En effet, outre son rôle crucial dans l'élevage du vin, ce récipient reste emblématique et riche d'une histoire qui rappelle de grandes épopées du commerce, et même de la science lorsqu'il a par exemple servi à nommer la célèbre expérience du « crève tonneau » ayant donné naissance au principe de Pascal en hydrostatique.

Pour être réutilisés dans de bonnes conditions, les tonneaux doivent être reconditionnés après un certain nombre d'utilisations. En effet, dans le cas de l'élevage du vin, l'apport aromatique des tonneaux diminue avec les vinifications consécutives.

Il existe de nombreux procédés de reconditionnement pour rendre les tonneaux usagés réutilisables. La plupart de ces procédés utilisent des nettoyages au jet d'eau suivis de traitements chimiques qui peuvent parfois occasionner la dissolution d'intrants dans le vin, au risque d'altérer la qualité du vin.

D'autres procédés utilisent l'étuvage par injection de vapeur sous pression. La vapeur se condense sur les parois internes du tonneau, ce qui permet à la fois d'élever leur température, au point de détruire les ferments, et de les rincer. Or, la vapeur a pour inconvénient de dégrader les composés aromatiques du bois et donc de compromettre l'élevage du vin.

D'autres procédés utilisant le sablage, le rabotage, le traitement par micro-ondes sont connus, mais peuvent assécher les tonneaux sans pour autant éliminer les bactéries qui colonisent les pores du bois en profondeur.

Le plus souvent, les différentes techniques précitées sont combinées pour obtenir un procédé de reconditionnement adapté aux spécificités de chaque type de tonneau.

Le document WO2008054707 décrit par exemple des procédés de reconditionnement d'un tonneau préalablement utilisé pour conserver une substance donnée, comprenant les étapes consistant à déterminer des paramètres de découpe spécifiques au matériau du tonneau pour découper de façon contrôlée le matériau du tonneau ; éliminer une profondeur désirée du matériau du tonneau en utilisant un dispositif de coupe automatisé à jet d'eau haute pression ; tester s'il reste des résidus de la substance en utilisant un dispositif de détection ; pulvériser éventuellement un agent dissolvant ; et répéter les étapes d'élimination et de test en cas de détection de la présence de la substance dans ou sur le tonneau.

On connaît également l'utilisation des ultrasons pour le nettoyage des tonneaux. Toutefois, les ultrasons seuls ne sont pas très efficaces pour le détartrage et nécessitent un important nettoyage préalable par action mécanique.

Le document FR2975312 décrit un procédé de nettoyage, de détartrage et de désinfection de tonneaux, comprenant un perçage d'un fond du tonneau, un nettoyage interne par jet d'eau chaude sous haute pression, une finition du nettoyage par des ultrasons et des ultraviolets germicides de type C, un rinçage suivi d'un égouttage, et un méchage avec du dioxyde de soufre (SO₂). Cette solution correspond à un nettoyage physique et chimique très poussé des parois, mais ne garantit pas un nettoyage en profondeur lorsque les parois sont bien pénétrées par le vin, car ne propose aucun retrait de matière sur lesdites parois.

### Présentation de l'invention

La présente invention vise à pallier les inconvénients de l'art antérieur, exposés ci-avant, en proposant une solution innovante pour un reconditionnement sans le moindre traitement chimique, économique et particulièrement écoresponsable.

À cet effet, la présente invention a pour objet un procédé de reconditionnement d'un tonneau usagé, notamment pour l'élevage du vin, comportant des douelles et deux fonds, les douelles assemblées formant une coque, ledit procédé comprenant :
- une étape de démontage des fonds pour isoler la coque ; et
- une étape de rabotage des faces internes de la coque pour retirer une couche pénétrée par des substances précédemment conservées dans le tonneau.

Ce procédé est remarquable en ce qu'il comprend, après les étapes précitées :
- une étape de chauffe de la coque ; et
- une étape de nettoyage des faces internes du tonneau par ultrasons.

Le procédé de reconditionnement selon l'invention permet ainsi d'augmenter la durée de vie des tonneaux traités tout en préservant leur potentiel organoleptique.

Avantageusement, les faces internes des fonds du tonneau peuvent également être rabotées.

Selon un mode de réalisation, le procédé comprend en outre une étape de remontage des fonds juste avant l'étape de nettoyage par ultrasons.

Plus particulièrement, l'étape de nettoyage par ultrasons peut être réalisée dans le tonneau remonté, par un trou de bonde dudit tonneau.

Selon un mode de réalisation particulier, l'étape de rabotage est automatisée et consiste à mettre en rotation la coque autour de son axe de révolution et à venir au contact des faces internes de ladite coque avec une tête raboteuse mobile suivant au moins deux axes.

De façon avantageuse, l'étape de nettoyage par ultrasons est réalisée avec des ultrasons haute puissance d'une fréquence de 20kHz par exemple. Selon l'invention, le procédé comprend en outre une étape finale de vaporisation de tanins sur les faces internes du tonneau, pour des raisons sanitaires, surtout lorsque le tonneau reconditionné est destiné à être transporté pendant de longues durées avant d'être rempli. Avantageusement, l'étape de chauffe et l'étape de nettoyage par ultrasons sont réalisées dans cet ordre pour éviter un démontage et un remontage supplémentaires.

Les concepts fondamentaux de l'invention venant d'être exposés dans leur forme la plus élémentaire, d'autres détails et caractéristiques ressortiront plus clairement à la lecture de la description qui suit et en regard des dessins annexés, donnant à titre d'exemple non limitatif un mode de réalisation d'un procédé de reconditionnement de tonneau par ultrasons conforme aux principes de l'invention.

### Présentation des dessins

Les figures sont données à titre purement illustratif pour une meilleure compréhension de l'invention sans en limiter la portée. Les différents éléments sont représentés de manière schématique. Sur l'ensemble des figures, les éléments identiques ou équivalents portent la même référence numérique.

Il est ainsi illustré en :
•[Fig. 1] : une vue en perspective d'un tonneau pour la mise en œuvre de l'invention ;
• [Fig. 2] : un synoptique des principales étapes d'un procédé de reconditionnement de tonneau par ultrasons selon un mode de réalisation de l'invention ;
• [Fig. 3] : l'étape de démontage des fonds ;
• [Fig. 4] : l'étape de rabotage automatique de la coque ;
• [Fig. 5] : l'étape de chauffe du tonneau ;
• [Fig. 6] : l'étape de nettoyage du tonneau par ultrasons ;
• [Fig. 7] : l'étape de vaporisation de tanins.

### Description détaillée de modes de réalisation

Dans le mode de réalisation décrit ci-après, on fait référence à un procédé de reconditionnement de tonneaux par ultrasons, destiné principalement aux barriques d'élevage du vin. Cet exemple, non limitatif, est donné pour une meilleure compréhension de l'invention et n'exclut pas l'utilisation d'un tel procédé de reconditionnement sur d'autres contenants en bois, notamment divers fûts en bois.

Dans la présente description, le terme « tonneau » désigne un récipient en bois à symétrie de révolution, renflé en son milieu, constitué de douelles cintrées assemblées et maintenues par des cercles généralement métalliques, fermé par deux fonds plats, et servant à conserver/élever et à transporter des liquides de consommation, en particulier des boissons alcoolisées telles que le vin.

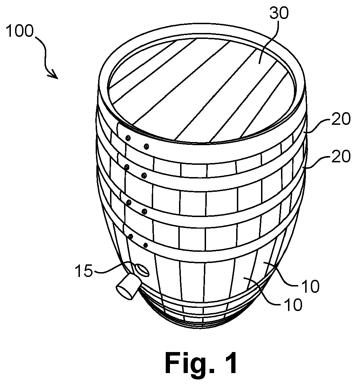

La figure 1 représente un tonneau 100 « traditionnel », adapté à la mise en œuvre de l'invention, comportant une pluralité de douelles 10 assemblées par des cercles 20 rivetés et deux fonds 30, dont seul le fond supérieur est visible sur la figure. Le tonneau 100 peut être rempli et vidé par un trou de bonde 15 ménagé dans sa douelle maîtresse.

Le tonneau 100, lorsqu'il est vidé de son contenu (vin par exemple), doit être nettoyé afin d'éviter l'apparition de moisissures sur ses faces internes, en particulier de bactéries acétiques. De plus, après un certain nombre de vinifications, il est nécessaire de totalement reconditionner le tonneau 100 pour le rendre réutilisable. En effet, dans le cas de l'élevage du vin, les vinifications répétées dans un même tonneau atténuent fortement, voire suppriment, les échanges entre le bois et le vin, qui sont très importants dans le processus d'élaboration du vin.

À cet effet, le tonneau 100 peut être reconditionné par la mise en œuvre d'un procédé de reconditionnement par ultrasons selon l'invention.

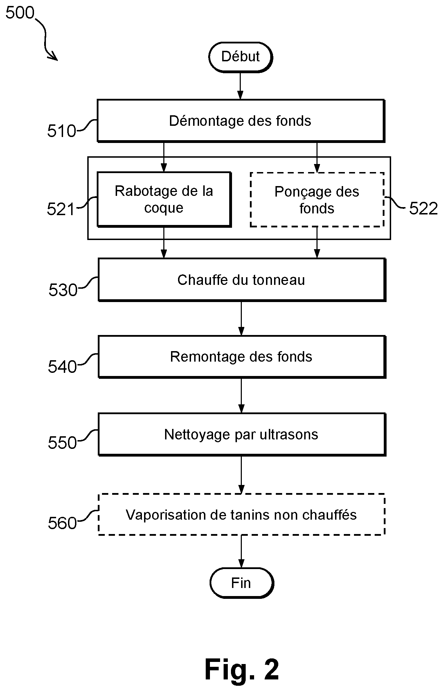

La figure 2 représente les principales étapes d'un procédé de reconditionnement 500 d'un tonneau, ledit procédé comprenant :
- une étape initiale 510 de démontage des fonds pour isoler la coque ;
- une étape 521 de rabotage des faces internes de la coque ;
- une étape 522, optionnelle, de ponçage des fonds pour une meilleure finition ;
- une étape 530 de chauffe du tonneau ;
- une étape 540 de remontage des fonds pour reconstituer le tonneau ;
- une étape 550 de nettoyage du tonneau par ultrasons ; et
- une étape 560, de vaporisation de tanins à l'intérieur du tonneau.

Bien entendu, le procédé de reconditionnement 500 est mis en œuvre après un rinçage, un égouttage et un séchage du tonneau, pour préparer les étapes de travail du bois, à savoir le rabotage et le ponçage.

L'étape 510 de démontage des fonds consiste à retirer les fonds pour isoler la coque, la coque étant constituée des douelles assemblées, et peut être réalisée par une méthode usuelle connue de l'homme du métier, en particulier des tonneliers. Cette séparation permet alors de traiter individuellement la coque et chaque fond lors des étapes 521 de rabotage des faces internes de la coque et 522 de ponçage des fonds.

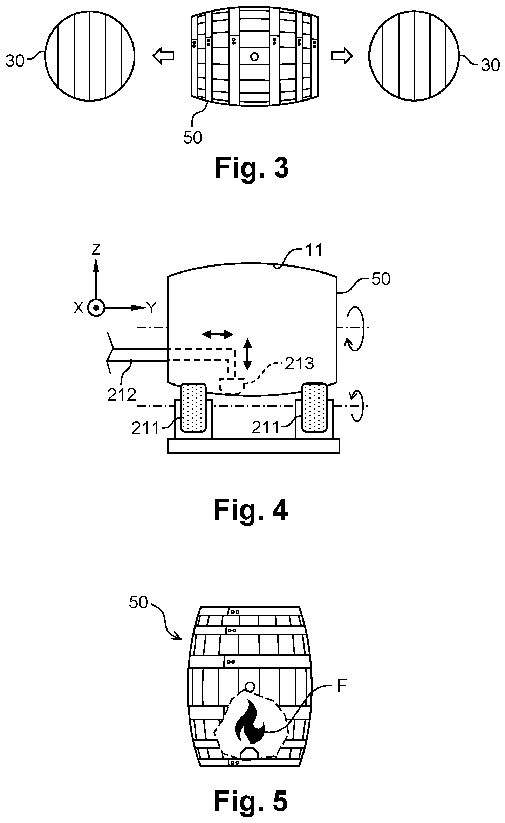

La figure 3 schématise l'étape 510 de démontage des fonds 30 pour isoler la coque 50.

Ensuite, l'étape 521 de rabotage des faces internes de la coque consiste à raboter la face interne de chaque douelle afin de retirer une couche pénétrée par le vin. Cela permet d'éliminer l'éthanol présent dans le bois avant le nettoyage par ultrasons.

Le rabotage peut être fait manuellement ou automatiquement au moyen de tout outil ou système adapté.

La figure 4 représente un mode de réalisation dans lequel l'étape 521 de rabotage de la coque 50 est réalisée automatiquement en positionnant ladite coque sur des moyens de mise en rotation 211 actionnés par des moteurs et en introduisant un bras mobile 212 pourvu d'une tête raboteuse 213 à l'intérieur de la coque pour venir au contact des faces internes 11 des douelles. La tête raboteuse 213 est de préférence orientée vers le bas pour bénéficier de l'effet de la gravité lors du rabotage. Ce système automatisé de rabotage peut être commandé par un automate programmable permettant de régler la vitesse de rotation des coques, la position du bras et d'autres paramètres fonctionnels. Selon cet exemple, la coque 50 est mise en rotation autour de son axe de révolution, parallèle à un axe horizontal Y du système d'axes (X,Y,Z) représenté. Le bras de rabotage 212 est quant à lui mobile en translation suivant l'axe horizontal Y et l'axe vertical Z. Ainsi, ces deux mouvements de translation de la tête raboteuse 213 permettent de couvrir toute la longueur de la coque 50 et d'épouser le renflement de celle-ci respectivement.

Le rabotage se fait alors par bandes circonférentielles successives sur toute la longueur de la coque 50.

Bien entendu, le rabotage des faces internes des douelles peut se faire différemment, par exemple en démontant les douelles et en les rabotant individuellement au moyen d'un rabot traditionnel.

Le rabotage peut également concerner les faces internes des fonds 30 dans certains cas, notamment lorsque les tonneaux reconditionnés seront destinés au vin blanc après qu'ils aient servi à élever du vin rouge.

Toutefois, le non-rabotage des fonds 30 est préféré pour conserver une pellicule tartrique et éviter que le vin soit en contact avec du bois vierge.

L'étape 522 de ponçage des fonds 30 est optionnelle et consiste à poncer la face externe de chaque fond 30 pour éliminer les traces d'usure et obtenir une meilleure finition du tonneau reconditionné. Cette étape peut être réalisée à tout moment du procédé.

Le rabotage peut concerner aussi bien des douelles 10 que des fonds 30 en raison de la pénétration du vin dans quasiment toutes les parois internes du tonneau. En fonction de la position de stockage du tonneau, verticale ou horizontale, la pénétration du vin dans le bois peut être plus importante soit dans des douelles soit dans le fond inférieur dudit tonneau.

L'étape 521 de rabotage constitue ainsi un premier nettoyage (décapage) du tonneau par retrait de matière, qui permet d'enlever les dépôts résiduels mais également les couches de bois pénétrées par l'éthanol.

Néanmoins, des moisissures peuvent subsister en profondeur dans les pores du bois raboté.

Les étapes qui suivent permettent donc de désinfecter le tonneau en profondeur.

Avant de remonter le tonneau pour le nettoyer par ultrasons, il est plus judicieux de chauffer la coque pour éviter de redémonter les fonds et de les remonter une fois de plus. De ce fait, il est préférable de réaliser les étapes 530 de chauffe, 540 de remontage des fonds et 550 de nettoyage par ultrasons dans cet ordre.

La figure 5 schématise l'étape 530 de chauffe de la coque 50 qui permet de « cuir » les faces internes des douelles à l'aide d'une flamme ou d'une braise F afin d'assouplir la fibre de bois et de regénérer son potentiel organoleptique. En effet, l'étape de chauffe est importante dans la mesure où l'étape 510 de démontage des fonds et l'étape 521 de rabotage de la coque ont pour conséquence d'annihiler les effets de la chauffe initiale ayant accompagnée la fabrication du tonneau.

L'étape 540 de remontage des fonds consiste à replacer les fonds 30 sur la coque 50 chauffée pour permettre le nettoyage du tonneau reconstitué par ultrasons.

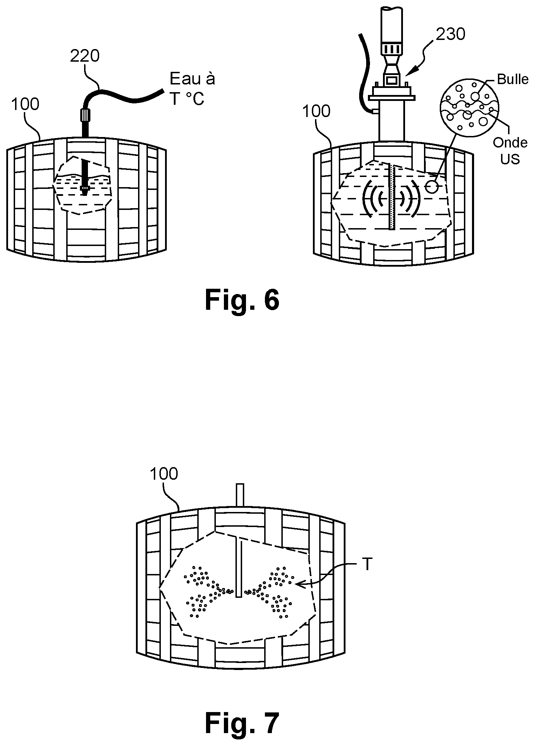

La figure 6 schématise l'étape 550 de nettoyage par ultrasons qui consiste dans un premier temps à remplir entièrement le tonneau 100 d'eau chaude et, dans un deuxième temps, à générer des ondes ultrasonores dans l'eau pour créer un phénomène de cavitation.

Selon l'exemple de réalisation illustré, le remplissage du tonneau 100 se fait par un moyen de remplissage 220 adapté, tel qu'une lance ou un tuyau raccordé à un réservoir d'eau chaude, introduit dans le trou de bonde.

L'eau de remplissage peut par exemple être à une température d'environ 60°C et sous pression pour favoriser sa pénétration dans les pores du bois.

L'eau chaude de remplissage peut avoir un effet nettoyant préliminaire, en particulier sur des corps gras qui s'émulsionnent et se désagrègent plus facilement sous l'effet de la chaleur.

Après le remplissage du tonneau 100, un nettoyage par ultrasons est réalisé au moyen d'un système adapté comprenant un générateur (non représenté) et un transducteur 230.

De préférence, les ondes ultrasonores générées sont de haute puissance. Ces ondes se propagent dans l'eau de remplissage du tonneau et créent des bulles microscopiques, dont les diamètres sont de l'ordre du micromètre. C es bulles sont ensuite excitées par les mêmes ondes ultrasonores US progressives et éclatent en générant des turbulences qui permettent de décomposer par action mécanique les corps indésirables se trouvant à proximité.

Ce phénomène de cavitation permet par exemple de briser les cristaux de tartre, d'éclater les parois cellulaires des micro-organismes et d'extirper les traces de vin résiduelles contenues dans les profondeurs des pores du bois. Plus la puissance des ondes ultrasonores est élevée plus l'action mécanique de nettoyage est efficace.

Ainsi, l'étape 550 de nettoyage par ultrasons apporte une action de détartrage, de désinfection et d'aseptisation des faces internes du tonneau.

Alternativement, la chauffe peut être réalisée après le nettoyage pour permettre surtout de regénérer le potentiel organoleptique apporté par le tonneau et donc de préserver la qualité du vin qui sera élevé dans le tonneau reconditionné.

Néanmoins, l'effet du nettoyage ultrasonore sur le potentiel organoleptique reste négligeable et ne justifie pas l'inversion des étapes de chauffe et de nettoyage ultrasonore, d'autant plus que cette inversion nécessite un démontage et un remontage supplémentaires des fonds.

En effet, l'ordre des étapes représenté en figure 2 correspond à une solution optimale obtenue par les inventeurs après différents essais dans lesquels différents ordres ont été testés. D'autres combinaisons restent toutefois possibles et couvertes par la présente invention dont l'objet principal est la combinaison du rabotage et du nettoyage par ultrasons.

La figure 7 schématise l'étape 560 de vaporisation de tanins, selon l'invention qui consiste à pulvériser des tanins liquides T non chauffés sur les parois internes du tonneau 100 au moyen d'un dispositif adapté tel qu'un atomiseur (spray).

Bien entendu, les tests gustatifs peuvent être réalisés uniquement sur un échantillon de tonneaux reconditionnés pour décider de la réalisation ou non de l'étape 560 de vaporisation de tanins sur les autres tonneaux.

## Revendications

1. Procédé (500) de reconditionnement d'un tonneau (100) usagé comportant des douelles (10) et deux fonds (30), les douelles assemblées formant une coque (50), ledit procédé comprenant : une étape (510) de démontage des fonds pour isoler la coque (50) ; une étape (521) de rabotage des faces internes (11) de la coque pour retirer une couche pénétrée par des substances précédemment conservées dans le tonneau ; et tel qu'il comprend, après les étapes (510) de démontage des fonds et (521) de rabotage des faces internes : une étape (540) de chauffe de la coque ; et une étape (550) de nettoyage des faces internes du tonneau par ultrasons, en ce que les faces internes des fonds (30) sont également rabotées, **caractérisé en ce que en ce que** ledit procédé comprend en outre une étape finale (560) de vaporisation de tanins sur les faces internes du tonneau (100) jusqu'à l'obtention des propriétés organoleptiques souhaitées.

2. Procédé selon la revendication 1, comprenant en outre une étape (530) de remontage des fonds (30) juste avant l'étape (550) de nettoyage par ultrasons.

3. Procédé selon la revendication 2, dans lequel l'étape (550) de nettoyage par ultrasons est réalisée dans le tonneau (100) remonté, par un trou de bonde (15) dudit tonneau.

4. Procédé selon l'une quelconque des revendications précédentes, dans lequel l'étape (521) de rabotage est automatisée et consiste à mettre en rotation la coque (50) autour de son axe de révolution et à venir au contact des faces internes de ladite coque avec une tête raboteuse (213) mobile suivant au moins deux axes.

5. Procédé selon l'une quelconque des revendications précédentes, lequel l'étape (550) de nettoyage par ultrasons est réalisée avec des ultrasons haute puissance d'une fréquence de 20kHz.

6. Procédé selon l'une quelconque des revendications précédentes, dans lequel l'étape (540) de chauffe et l'étape (550) de nettoyage par ultrasons sont réalisées dans cet ordre.

7. Tonneau reconditionné, obtenu par un procédé (500) de reconditionnement selon l'une des revendications précédentes.

## Patentansprüche

1. Verfahren (500) zur Wiederaufbereitung eines gebrauchten Fasses (100) mit Dauben (10) und zwei Böden (30), wobei die zusammengebauten Dauben eine Schale (50) bilden, wobei das Verfahren Folgendes umfasst: einen Schritt (510) des Demontierens der Böden, um die Schale (50) zu isolieren; einen Schritt (521) des Abschleifens der Innenseiten (11) der Schale, um eine Schicht zu entfernen, die von zuvor im Fass aufbewahrten Stoffen durchdrungen ist; und wie es nach den Schritten (510) des Demontierens der Böden und (521) des Abschleifens der Innenseiten Folgendes umfasst: einen Schritt (540) des Erhitzens der Schale; und einen Schritt (550) des Reinigens der Innenseiten des Fasses mit Ultraschall, wobei die Innenseiten der Böden (30) ebenfalls abgeschliffen werden, **dadurch gekennzeichnet, dass** das Verfahren ferner einen letzten Schritt (560) des Verdampfens von Tanninen auf den Innenseiten des Fasses (100) bis zum Erhalten der gewünschten organoleptischen Eigenschaften umfasst.

2. Verfahren nach Anspruch 1, ferner umfassend einen Schritt (530) zur Wiedermontage der Böden (30) unmittelbar vor dem Schritt (550) des Reinigens mit Ultraschall.

3. Verfahren nach Anspruch 2, wobei der Schritt (550) des Reinigens mit Ultraschall in dem wieder montierten Fass (100) durch ein Spundloch (15) des Fasses durchgeführt wird.

4. Verfahren nach einem der vorhergehenden Ansprüche, wobei der Schritt (521) des Abschleifens automatisiert ist und darin besteht, die Schale (50) um ihre Drehachse zu drehen und die Innenseiten der Schale mit einem beweglichen Abschleifkopf (213) entlang mindestens zweier Achsen in Kontakt zu bringen.

5. Verfahren nach einem der vorhergehenden Ansprüche, wobei der Schritt (550) des Reinigens mit Ultraschall mit Hochleistungsultraschall mit einer Frequenz von 20 kHz durchgeführt wird.

6. Verfahren nach einem der vorhergehenden Ansprüche, wobei der Schritt (540) des Erhitzens und der Schritt (550) des Reinigens mit Ultraschall in dieser Reihenfolge durchgeführt werden.

7. Wiederaufbereitetes Fass, erhalten durch ein Verfahren (500) zur Wiederaufbereitung nach einem der vorhergehenden Ansprüche.

## Claims

1. A method (500) for reconditioning a used cask (100) comprising staves (10) and two heads (30), the assembled staves forming a shell (50), said method comprising: a step (510) of disassembling the heads to isolate the shell (50); a step (521) of planing the inner faces (11) of the shell to remove a layer penetrated by substances previously preserved in the cask; and such that it comprises, after the steps (510) of disassembling the heads and (521) of planing the inner faces: a step (540) of heating the shell; and a step (550) of ultrasonically cleaning the inner faces of the cask, in that the inner faces of the heads (30) are also planed, **characterized in that** said method further comprises a final step (560) of vaporizing tannins on the inner faces of the cask (100) until the desired organoleptic properties are achieved.

2. The method according to claim 1, further comprising a step (530) of reassembling the heads (30) just before the ultrasonic cleaning step (550).

3. The method according to claim 2, wherein the ultrasonic cleaning step (550) is carried out in the cask (100) reassembled, by a bung hole (15) of said cask.

4. The method according to any one of the preceding claims, wherein the planing step (521) is automated and consists in rotating the shell (50) about its axis of revolution and coming into contact with the inner faces of said shell with a planing head (213) movable along at least two axes.

5. The method according to any one of the preceding claims, wherein the ultrasonic cleaning step (550) is performed with high-power ultrasound having a frequency of 20kHz.

6. The method according to any one of the preceding claims, wherein the heating step (540) and the ultrasonic cleaning step (550) are performed in this order.

7. A reconditioned cask, obtained by a reconditioning method (500) according to one of the preceding claims.
